# EUROPEAN PATENT APPLICATION

(11) **EP 1 709 986 A1**
(43) Date of publication of application: **11.10.2006**
(21) Application number: 06007129.7
(22) Date of filing: 04.04.2006
(51) Int. Cl.: A61M 25/00, A61F 2/06

(54) **Integrated detachable introducer**

(30) Priority: 08.04.2005 US 102494
(71) Applicant: Medtronic Vascular, Inc., Santa Rosa, California 95403 (US)
(72) Inventor: Huszar, Hilary, San Francisco California 94131 (US)
(74) Representative: Zimmermann, Gerd Heinrich

(57) **Abstract**

An apparatus is disclosed for an integrated detachable introducer (102) operable with a catheter assembly (104). The catheter assembly (104) includes a shaft (110), a handle (108) and an implantable therapeutic device (106) mounted on the shaft (110). The integrated detachable introducer (102) includes an introducer body (126) designed to releaseably couple with the handle (108), and an introducer sheath (124) that is coupled the introducer body (126). The integrated detachable introducer (102) further includes a lumen (108) extending through the introducer body (126) and the introducer sheath (124); the lumen is sized to slideably fit over the shaft (110) of the catheter delivery system (110). The integrated detachable introducer (102) is used to seal an incision to the blood vessel once the catheter delivery system has delivered the proper therapy or therapeutic device (106). If additional therapy is required, the blood vessel may be accessed through the integrated detachable introducer (102).

## Description

### FIELD OF THE INVENTION

The present invention relates generally to catheter delivery systems for the treatment of abdominal aortic aneurysms or other vascular diseases, especially those requiring large diameter delivery devices, and more particularly to an integrated detachable introducer used in conjunction with the catheter delivery system.

### BACKGROUND OF THE INVENTION

One method of treating aortic aneurysms involves invasive surgical procedures with graft placement within the vessel as a replacement for the affected native artery. Such a procedure requires a highly invasive surgical operation through the abdomen in order to access the aorta. Many patients are unable to receive this procedure due to poor physical condition, which would make the risk of mortality or co-morbities extremely high. In addition, this invasive surgical procedure has many negative outcomes, such as severe blood loss, long hospital stays, and renal damage.

Another method has been developed for treating aortic aneurysms that is non-invasive and includes a catheter delivery system having a graft that is inserted through the incision of a blood vessel and advanced to the affected vessel for deployment. These catheter delivery systems are usually too large to be used with any commercially available introducer, so physicians cut down to the entry blood vessel (e.g. femoral arteries) and directly access the vessel through a large incision (around 1 cm). After placement of the implantable therapeutic device, the delivery system is removed, leaving a large open access site. Often, physicians will try to quickly remove the delivery system and fill the incision with a secondary large bore introducer. Other times, physicians will try to quickly partially suture the incision and place a smaller introducer. These introducers are needed for the subsequent placement of other vascular devices, including additional implants, angiography catheters, and balloons. The process of having to remove the large delivery catheters and replace them with an introducer can lead to significant blood loss and poor patient outcomes.

Accordingly, it would be desirable to provide an integrated detachable introducer that is part of a catheter delivery system that can be slid into the incision of a blood vessel and left in place once the catheter assembly is removed. Furthermore, other desirable features and characteristics according to the present invention will become apparent from the subsequent detailed description and the appended claims, taken in conjunction with the accompanying drawings and the foregoing technical field and background.

### SUMMARY OF THE INVENTION

The present invention relates to an integrated detachable introducer that is used in conjunction with a catheter assembly. The integrated detachable introducer is used to seal the incision to the blood vessel once the catheter delivery system has delivered the proper therapy or implantable therapeutic device and has been removed. If additional therapy is required, the blood vessel may be accessed through the integrated detachable introducer.

An integrated detachable introducer is disclosed for use with a catheter assembly. The catheter assembly includes a shaft, a handle and an implantable therapeutic device mounted within the shaft. The integrated detachable introducer includes an introducer body that is designed to releaseably couple with the handle and an introducer sheath that is coupled to the introducer body. The integrated detachable introducer further includes a lumen extending through the introducer body and the introducer sheath; the lumen is sized to slideably fit over the shaft.

A catheter delivery system is disclosed that includes a catheter assembly and an integrated detachable introducer. The catheter assembly includes a shaft with proximal and distal ends, a handle coupled to the proximal end, and an implantable therapeutic device mounted on the distal end. The catheter delivery system further includes an integrated detachable introducer having a lumen therethrough, the lumen being sized to slideably fit over the shaft. The integrated detachable introducer is designed to releaseably couple to the handle.

A method is disclosed for using a catheter delivery system that includes a catheter assembly and an integrated detachable introducer. The catheter assembly includes a shaft, a handle and a therapeutic device. The integrated detachable introducer includes an introducer sheath sized to slideably fit over the shaft and designed to releaseably couple to the handle. The method includes making an incision to access a blood vessel and inserting the implantable therapeutic device into the blood vessel through the incision. The implantable therapeutic device is then positioned proximate the treatment site and therapeutic treatment is provided. The integrated detachable introducer is then uncoupled from the handle and advanced along the shaft with the introducer sheath inserted into the incision. The catheter delivery system is then withdrawn out through the integrated detachable introducer.

### BRIEF DESCRIPTION OF THE DRAWINGS

The following drawings are illustrative of particular embodiments according to the invention and therefore do not limit its scope. They are presented to assist in providing a proper understanding of the invention. The drawings are not to scale and are intended for use in conjunction with the explanations in the following detailed descriptions. Like reference numerals denote like elements in the drawings, wherein;

FIG. 1 is a side view of a catheter delivery system with a catheter assembly and an integrated detachable introducer;

FIG. 2 is a perspective view showing the integrated detachable introducer mated with the handle of the catheter assembly;

FIG. 3 is a longitudinal cross-sectional view of the integrated detachable introducer mated with the handle of the catheter assembly, as viewed from line 3-3 in FIG. 2;

FIG. 4 is an enlarged side view of the handle area of the catheter delivery system as viewed in dashed circle 4 in FIG. 1;

FIG. 5 is the side view of FIG. 4 showing the separation of the integrated detachable introducer separating from the handle of the catheter assembly; and

FIG. 6 is a rear perspective view of the integrated detachable introducer showing details of the integrated detachable introducer.

### DETAILED DESCRIPTION

The following detailed description of the invention is merely exemplary in nature and is not intended to limit the invention or the application and/or uses of the invention. Furthermore, there is no intention to be bound by any theory presented in the preceding background of the invention or the following detailed description of the invention.

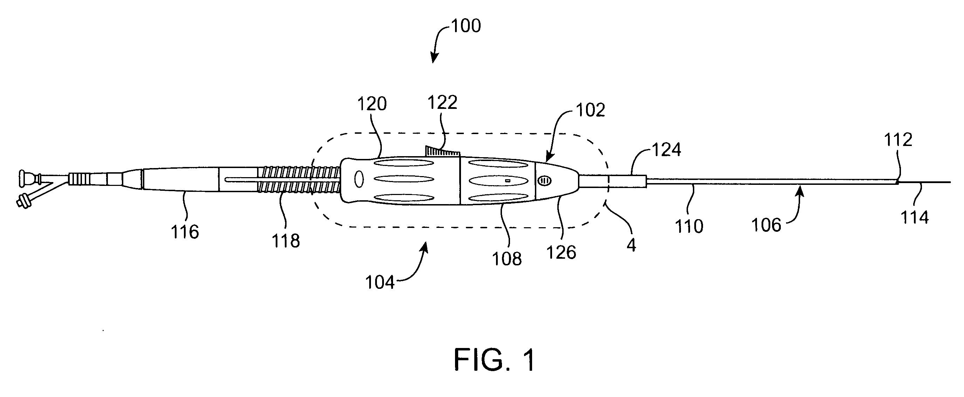

FIG. 1 shows a catheter delivery system 100 having an integrated detachable introducer 102 mounted on a catheter assembly 104. The catheter assembly 104 includes an implantable therapeutic device 106, such as a self-expanding stent graft, that is used for the treatment of abdominal aortic aneurysms or other vascular diseases, especially those requiring large diameter delivery devices. The catheter assembly 104 is usually a single use, disposable device with the implantable therapeutic device 106 mounted on a distal end of the catheter assembly 104. The catheter assembly 104 also includes an integrated handle 108 to provide the user with controlled implant deployment. The catheter assembly 104 is flexible and is compatible with a guidewire (not shown), such as a 0.889 mm (0.035") guidewire.

The catheter assembly 104 may be any conventional therapy delivery system, such as the Talent AAA Stent Graft with the Xcelerant Delivery System, sold by Medtronic, Inc. The catheter assembly 104 may include a shaft 110 having proximal and distal ends, one or more radio opaque markers 112 on the shaft, a tapered tip 114 at the distal end of the shaft, a rear grip 116, a screw gear 118, a slider 120 and a trigger 122. A handle 108 is on the proximal end of the shaft 110 and the implantable therapeutic device 106 is mounted on the distal end of the catheter assembly 104. The catheter shaft 110 may include up to three concentric single lumen polymer shafts, including an outer cover covering the implantable therapeutic device 106, an inner member shaft upon which the implantable therapeutic device 106 is mounted, and a guidewire lumen through which a guidewire may be inserted. Various o-rings may be contained within the catheter assembly 104 to maintain hemostasis and prevent blood loss and leaking during the procedure. Retraction of the cover on shaft 110 allows deployment of the implantable therapeutic device 106, such as a self-expanding stent graft. Post deployment, the physician will recapture the tapered tip 114 of the catheter assembly 104 by retracting an inner member of the catheter assembly 104.

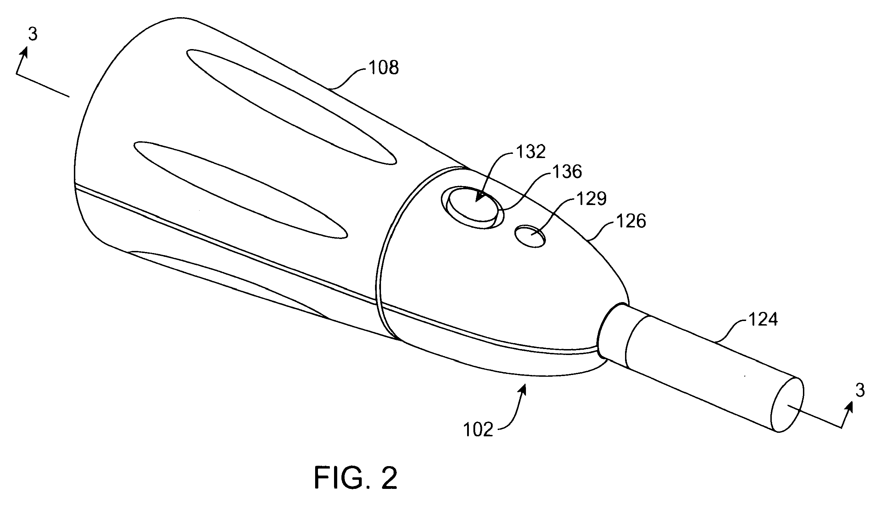

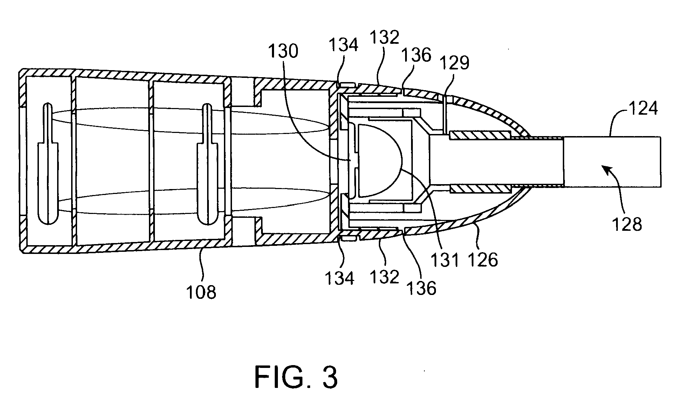

FIG. 2 is a perspective view and FIG. 3 is a longitudinal cross-sectional view showing the integrated detachable introducer 102 including an introducer portion or introducer sheath 124, a body portion 126, a lumen 128 extending through the introducer portion 124 and the body portion 126 and a flush port 129. One or more hemostatic valves may be used in fluid communication with the lumen 128. Shown are two passive lock valves, a slotted release valve 130 and a dome valve 131, positioned in series. The slotted release valve 130 seals on guidewires and then fails when a larger device is pushed through it. The dome valve 131 is then used to seal on the large devices. While the two passive lock valves have been shown, other valves or seals may be used, such as touhy borst seal, iris valve, or air bladder.

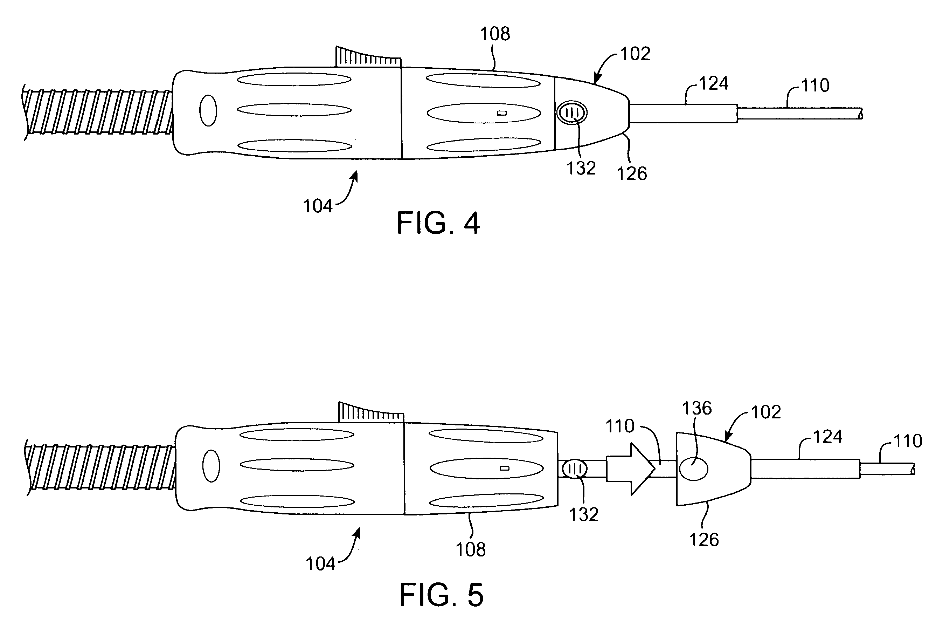

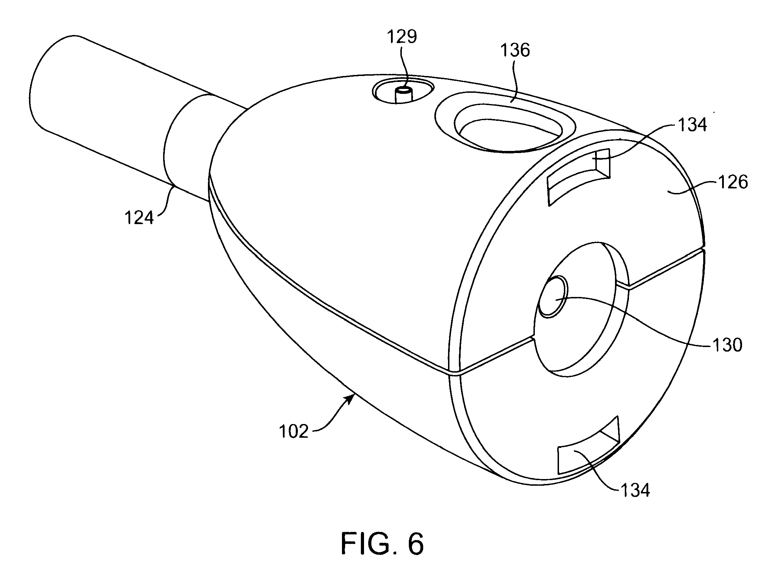

FIG. 4 is an enlarged side view of the handle area of the catheter delivery system of FIG. 1. The lumen 128 is sized to slideably fit over the shaft 110 of the catheter assembly 104 and slide up to the handle 108. To slide, the lumen 128 is usually sized 2 French sizes larger than the shaft 110. The handle 108 includes one or more lever (locking) arms or release buttons 132. In the embodiment shown, two opposing release buttons 132 are shown in the handle 108, but one button will also work. The body portion 126 includes a slot 134 configured to connect to an opening 136, one for each release button 132 (Fig. 6). In another configuration, the release buttons 132 can be attached to the introducer body portion 126 and the release slots 134 are located in the handle 108. To join the integrated detachable introducer 102 to the handle 108, each release button 132 is depressed slightly and inserted into slot 134. When the release button 132 is fully inserted into the slot 134, the release button 132 pops up into opening 136, locking the integrated detachable introducer 102 to the handle 108. To release the integrated detachable introducer 102 from the handle 108, the release buttons 132 are depressed simultaneously, such as with the index finger and thumb, unlocking or detaching the integrated detachable introducer 102 from the handle 108. Once released, the integrated detachable introducer 102 is then free to slide relative to the shaft 110, separating it from the handle 108, such as shown in FIG. 5. The handle 108 and body portion 126 may be made of any suitable medical grade plastic, for example nylon. The introducer portion 124 may be of any suitable medical grade plastic, for example polyether block amide copolymer (pebax).

While release buttons 132 with mating slots 134 (and latching openings) have been shown, other types of connections which allow the integrated detachable introducer 102 to connect and disconnect from the handle 108 are contemplated. For example, the integrated detachable introducer 102 may be threaded onto the handle 108, so that it could screw on to attach and screw off to release. In another example, the integrated detachable introducer 102 and handle 108 could include magnetic portions, so that the introducer 102 could be magnetically attached and released from the handle 108.

FIG. 6 is a rear perspective view showing details of the integrated detachable introducer 102 alone, including the introducer portion 124, the body portion 126 and the valve 130. This also provides a view of the slots 134 into which the handle release buttons 132 are inserted for locking.

In use, the physician makes an incision in the access blood vessel and inserts the catheter assembly 104 of the catheter delivery system 100 into the access blood vessel. The catheter assembly 104 is then advanced until the implantable therapeutic device 106 is positioned proximate to the treatment site, where the implantable therapeutic device 106 is deployed. If there is an outer cover, the deployment of the implantable therapeutic device 106 may include withdrawing the outer cover to release the implantable therapeutic device 106. Following deployment/placement of the implantable therapeutic device 106, the physician disconnects and detaches the integrated detachable introducer 102 from the handle 108 by depressing release buttons 132 and sliding the integrated detachable introducer 102 relative to (e.g., forward along) the shaft 110. The integrated detachable introducer 102 can then be slid forward until the introducer portion 124 is inserted into the vessel incision (it is not already so positioned). The physician then pulls the catheter assembly 104 out through the integrated detachable introducer 102, leaving the integrated detachable introducer 102 behind so that a seal is formed upon removal of the delivery device. This configuration eliminates almost all blood loss and allows the physician to re-access the vessel easily through the integrated detachable introducer 102 for any needed secondary procedures.

Once in place, the integrated detachable introducer 102 is used to provide endovascular access to the access blood vessel to facilitate insertion of additional catheters into and manipulation within the blood vessel without requiring a separate vessel puncture for each catheter. The integrated detachable introducer 102 has a relatively short tubular sheath or introducer portion 124 (approximately 15 cm), open at its distal end (the end within the patient), with the body portion 126 attached to the proximal end (outside of the patient) of the sheath. The body portion 126 typically includes a proximal aperture through which a catheter can be passed as well as a self-sealing valve or gasket, such as valves 130, 131, to effect a seal to prevent blood from leaking out of the integrated detachable introducer 102, both when a catheter is passed through the integrated detachable introducer 102, as well as when the catheter assembly 104 has been withdrawn from the integrated detachable introducer 102. The body portion 126 also may include a flush port or side port 129 coupled to the lumen 128 distal to the sealing valves through which blood may be sampled or medication or other physiological liquids may be introduced.

While exemplary embodiments have been presented in the foregoing detailed description, it should be appreciated that a vast number of variations exist. It should also be appreciated that the exemplary embodiment or exemplary embodiments are only examples, and are not intended to limit the scope, applicability, or configuration of the invention in any way. Rather, the foregoing detailed description will provide those skilled in the art with a convenient roadmap for implementing an exemplary embodiment of the invention, it being understood that various changes may be made in the function and arrangement of elements described in an exemplary embodiment without departing from the scope of the invention as set forth in the appended claims.

## Claims

1. An integrated detachable introducer (102) for use with a catheter assembly (104), the catheter assembly (104) having a shaft (110), a handle (108) and an implantable therapeutic device (106) mounted on the shaft (110), the integrated detachable introducer (102) comprising:
an introducer body (126) having a proximal end and a distal end, the proximal end being configured to releaseably couple with the handle (108);
an introducer sheath (124) coupled to the distal end of the introducer body (126); and
a lumen (128) extending through the introducer body (126) and the introducer sheath (124), the lumen (128) being sized to slideably fit over the shaft (110).

2. The introducer of claim 1, further comprising one or more hemostatic valves (130, 131) coupled to the lumen (128), the one or more hemostatic valves (130, 131) being configured to seal the lumen (128).

3. The introducer of claim 1 or 2, further comprising a flush port (129) in fluid communication with the lumen (128), the flush port (129) being configured to withdraw fluids from or introduce fluids into the lumen (128).

4. The introducer of claim 3, wherein the flush port (129) is positioned within the introducer body (126).

5. The introducer according to any of the preceding claims, wherein the handle (108) includes one or more release buttons (132) and the introducer body (126) includes one or more slots (134) configured to releaseably couple with the one or more release buttons (132).

6. The introducer of claim according to any of the preceding claims, wherein the introducer body (126) is formed from nylon.

7. The introducer of claim according to any of the preceding claims, wherein the introducer sheath (124) is formed from polyether block amide copolymer (pebax).

8. A catheter delivery system (100) comprising:
a catheter assembly (104) having a shaft (110) with proximal and distal ends, a handle (108) coupled to the proximal end of the shaft (110) and an implantable therapeutic device (106) mounted on the distal end of the shaft (110); and
an integrated detachable introducer (102) having a lumen (128) therethrough, the lumen (128) sized to slideably fit over the shaft (110), the integrated detachable introducer (102) being configured to releaseably couple to the handle (108).

9. The system of claim 8, the integrated detachable introducer (102) further including:
an introducer body (126) having a proximal end and a distal end, the proximal end of the introducer body (126) being configured to releaseably couple to the handle (108); and
an introducer sheath (124) coupled to the distal end of the introducer body (126);
wherein the lumen (128) extends through the introducer body (126) and the introducer sheath (124).

10. A catheter delivery system (100) comprising:
a catheter assembly (104) having a shaft (110) with proximal and distal ends, a handle (108) coupled to the proximal end of the shaft (110) and an implantable therapeutic device (106) mounted on the distal end of the shaft (110); and
an integrated detachable introducer (102) according to any of claims 1 to 7.

11. The system according to any of claims 8 to 10, wherein the shaft (110) includes an outer cover movably positioned over the therapeutic device (106), the outer cover being configured to move from a first position covering the implantable therapeutic device (106) to a second position uncovering the therapeutic device (106).

12. The system of claim 11, further comprising a slider (120) coupled to the outer cover, the slider (120) being configured to move the outer cover from the first position to the second position.

13. The system according to any of claims 8 to 12, wherein the implantable therapeutic device (106) is a stent graft.

14. The system according to any of claims 8 to 13, further comprising one or more radio opaque markers (112) coupled to the shaft (110).
